# EUROPEAN PATENT SPECIFICATION

(11) **EP 2 630 906 B1**
(45) Date of publication and mention of the grant of the patent: **31.10.2018**
(21) Application number: 13156287.8
(22) Date of filing: 22.02.2013
(51) Int. Cl.: A61B 1/00, A61B 1/04, A61B 1/313

(54) **Surgical support assembly**
Chirurgische Stützanordnung
Ensemble de support chirurgical

(30) Priority: 23.02.2012 US 201261602103 P; 04.02.2013 US 201313758156
(43) Date of publication of application: 28.08.2013
(73) Proprietor: Covidien LP, Mansfield, MA 02048 (US)
(72) Inventor: Viola, Frank, Sandy Hook, CT Connecticut 06482 (US); Prescott, Michael, Hamden, CT Connecticut 06514 (US)
(74) Representative: Soames, Candida Jane

(56) References cited:
- US-A1- 2007 255 100
- US-A1- 2008 309 758
- US-A1- 2010 152 539
- US-A1- 2011 257 488

## Description

### CROSS REFERENCE TO RELATED APPLICATION

The present application claims the benefit of and priority to U.S. Provisional Application Serial No. 61/602,103, filed on February 23, 2012.

### BACKGROUND

### 1. Technical Field

This application generally relates to the field of minimally invasive procedures. More particularly, the present disclosure relates to a surgical support assembly for positioning and securing a camera or projector inside the body in the proximity of a surgical site.

### 2. Background of Related Art

Today, many surgical procedures are performed through small incisions in the skin, as compared to the larger incisions typically required in traditional procedures, in an effort to reduce both trauma to the patient and recovery time. Generally, such procedures are referred to as endoscopic, unless performed on the patient's abdomen, in which case the procedure is referred to as laparoscopic. Throughout the present disclosure, the term minimally invasive should be understood to encompass both endoscopic and laparoscopic procedures. During a typical minimally invasive procedure, surgical objects, such as surgical access ports (e.g., trocar and/or cannula assemblies), endoscopes, or other instruments, are inserted into the patient's body through the incision in tissue. Prior to the introduction of the surgical object into the patient's body, insufflation gases may be used to enlarge the area surrounding the target surgical site to create a larger, more accessible work area.

As surgical objects are inserted into the patient's body, the problem of visibility of the insufflated workspace arises. Due to the nature of minimally invasive procedures, the relatively small incisions into tissue do not afford an operator much direct visibility into an insufflated workspace. Surgical imaging equipment is thus increasingly relied upon to visualize an insufflated workspace during minimally invasive procedures.

However, the presence of multiple tissue layers and surgical objects can obscure the field of view for an operator using surgical imaging equipment inserted through tissue. Further, imaging equipment that is inserted into a body cavity may be forced out of alignment due to the interactions of surgical objects and tissue within the insufflated workspace. Therefore, it is desirable to have imaging equipment that can be attached to a portion of a body cavity to view a surgical site above or below the equipment. Accordingly, a need exists for a device that can support imaging equipment in an insufflated workspace with the ability to adapt to changing conditions at the minimally invasive surgical site.
An apparatus for mounting a camera on a body cavity inner wall is known from US 2008/0309758.

### SUMMARY

The invention is defined by the appended claims. The present disclosure is directed toward a support assembly for an imaging member above a surgical site. The support assembly includes a plurality of support members that penetrate a layer of tissue and extend into a body cavity below. The support members may be rigid or resilient rods having a variety of shapes and cross-sectional profiles. In embodiments, the support members may be hollow to receive wires or other equipment.

Disposed on the surface of the layer of tissue through which the support members are inserted are position control members which control the depth of insertion of the support members. The position control members are generally flat members that receive the support members. The position control members may additionally control the degree to which the support members may be pivoted within the position control members. In embodiments, the position control members may include a rotatable member or other structure to assist the support members in pivoting relative to the position control member.

In other embodiments, the position control members may be flat pads through which the support members are inserted. The flat pads frictionally engage the outer surface of the support members and inhibit the amount of axial translation of the support members into an internal body cavity.

The position control members may incorporate a locking mechanism to secure the support members in place. The locking member may be a set screw or a latching element that engages a side surface of a support member to hold the support member in place to inhibit axial translation of the support member and may additionally inhibit the support member from pivoting within the position control member. In embodiments, separate locking mechanisms may be present for inhibiting axial translation and pivoting of the support members.

An imaging member, which may include a camera, a projector or an illuminator or a combination thereof, is attached at the distal portions of the support members to at a vertex of the support members. The secure attachment of the imaging element to the support members may include a threaded connection. In other embodiments, the coupling of the imaging member to the support members may be formed with an interference fit, tongue-and-groove, or bayonet-type connection. The imaging member collects images from the surgical site below. In embodiments, the imaging element may incorporate a projector, with collected or stored images projected onto a surface such as the interior surface of a tissue wall.

These and other embodiments of the present disclosure will be described in greater detail below with reference to the appended figures.

### BRIEF DESCRIPTION OF THE DRAWINGS

The accompanying drawings, which are incorporated in and form part of the specification, illustrate the present disclosure when viewed with reference to the description, wherein:
FIG. 1 is a perspective view of a surgical support assembly including three support members, three position control members, and an imaging member;
FIG. 2 is a partial assembly view of the surgical support assembly as shown in FIG. 1, with the support members inserted through the position control members and separate from the imaging member;
FIG. 3A is a side cross-sectional view of a position control member before a support member is inserted therethrough;
FIG. 3B shows the side cross-sectional view of FIG. 3A, with a support member partially inserted therethrough and pivoted relative to the position control member;
FIG. 3C shows the side cross-sectional view of FIG. 3A, with a support member fully inserted therethrough at an angle and locked into position;
FIG. 3D shows the side cross-sectional view of FIG. 3C, further including a passageway through which a set screw may directly engage the support member;
FIG. 4 is a cross-sectional view of the surgical support assembly of FIG. 1, fully assembled and disposed in a body cavity;
FIG. 5 is a side perspective view of an embodiment of a position control member as a flat pad, with a support member inserted therethrough;
FIG. 6 is a side cross-sectional view of partial assembly view of a surgical support assembly having two support members, with one support member securely attached to the imaging element with an interference fit, and another support member inserted into position in the body cavity prior to attachment to the imaging element; and
FIG. 7 is a bottom perspective view of an embodiment of a surgical support assembly having an imaging element incorporating a projector to display an image inside a body cavity.

Other features of the present disclosure will become apparent from the following detailed description taken in conjunction with the accompanying drawings, which illustrate, by way of example, the principles of the present disclosure.

### DETAILED DESCRIPTION OF EMBODIMENTS

The present disclosure will now describe in detail embodiments of a surgical support assembly with reference to the drawings in which like reference numerals designate identical or substantially similar parts in each view. Throughout the description, the term "proximal" will refer to the portion of the assembly closest to the operator, whereas the term "distal" will refer to the portion of the assembly farthest from the operator.

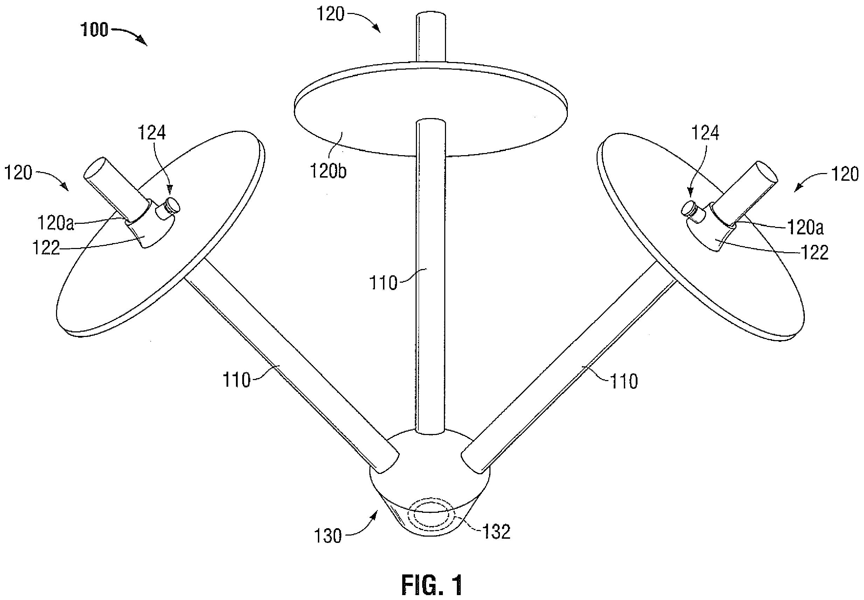

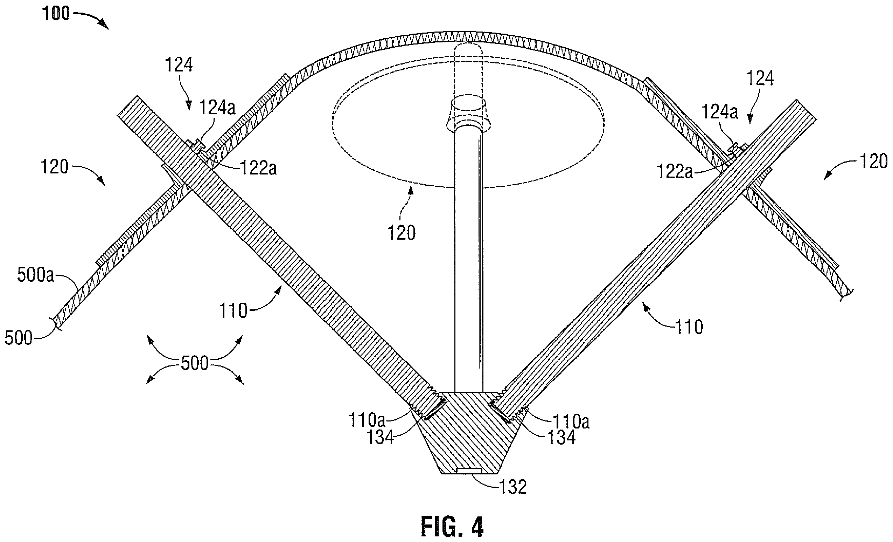

Referring initially to FIG. 1, a surgical support assembly 100 is shown fully assembled. The surgical support assembly 100 includes a plurality of support members 110 that penetrate a layer of tissue 500 (FIG. 4). The support members may have tips configured and dimensioned to cut through tissue (not shown), or may be inserted into pre-formed incisions in tissue. The support members 110 are generally elongate members that have a cylindrical cross-sectional profile. Other shapes with different cross-sectional profiles are contemplated for the support members 110. The support members 110 may be solid or hollow.

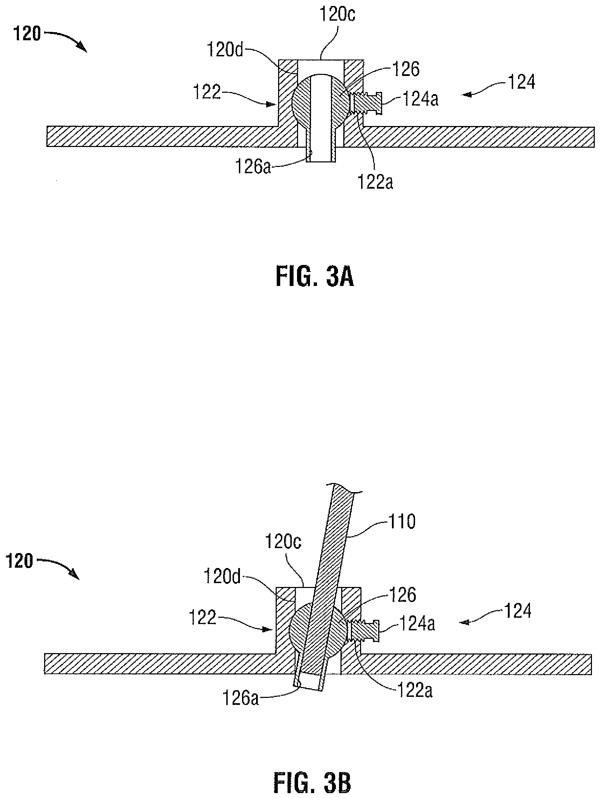

The support members 110 are inserted through respective position control members 120. Position control members 120 generally include a flat underside 120b that contacts a body surface 500a (FIG. 4). Underside 120b of position control members 120 may be otherwise shaped or curved to accommodate a variety of body surface conditions. Position control members 120 also include a receiving portion 122 that may be a neck or other protruding shape from the flat profile of position control member 120. At the proximal end 120a of the position control member 120 is an aperture 120c (FIG. 3A) that defines a passage 120d (FIG. 3A) through the position control member 120 through which a support member 110 may be inserted.

Position control members 120 may also include a locking mechanism 124 that affects movement of the support members 110 at least axially through the position control member 120 and layer of tissue 500. Locking mechanisms 124 can be used to maintain position control members 124 at a desired depth in a layer of tissue 500. The position control members 120 may also control the degree to which the support members 110 are free to pivot relative to the position control members 120, as will be described further below.

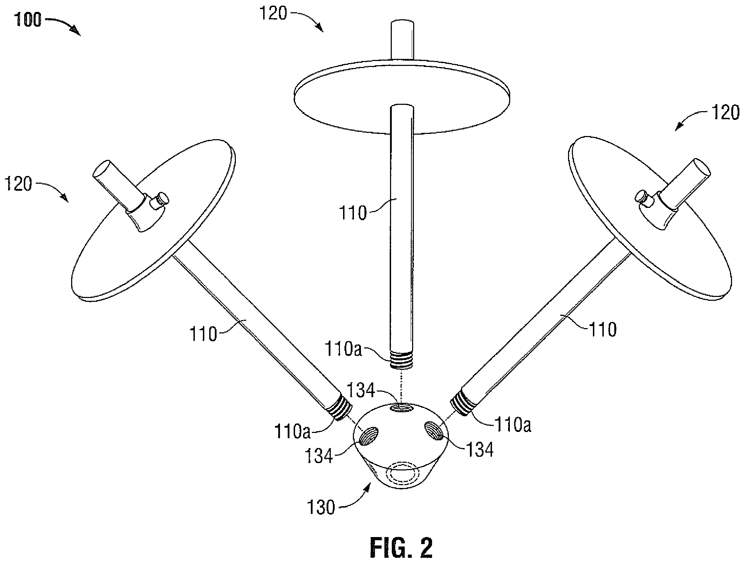

Attached to the distal portions 110a (FIG. 2) of the support members 110 is an imaging member 130. Imaging member 130 may have a pod-like shape as shown, though other shapes and profiles are contemplated for imaging member 130. The imaging member 130 incorporates an imaging aperture 132 (shown in phantom view) for collecting or projecting images in an internal body cavity 500b (FIG. 4). The imaging aperture 132 may be include a camera, a light pipe with polished ends, a fiber optic element, or a projector. The imaging member 130 is attached at the distal portions 110a of the support members 110 such that it forms an apex of the support members 110. The imaging aperture 132 is disposed on or embedded in a side surface of imaging member 130 such that it has a substantially unobstructed view of a surgical site. The positioning of the support members 110 and the imaging member 130 may also be inverted such that the imaging member is at a distal vertex and the imaging member looks proximally toward the abdominal wall, for example in a hernia operation.

Turning to FIG. 2, the surgical support assembly 100 is shown partially assembled, with the support members 110 inserted through the position control members 120, but separate from the imaging member 130. The imaging member 130 contains threaded receiving apertures 134 and the distal portions 110a of the support members 110 are threaded such that the support members 110 and the imaging member 130 threadably engage upon inserting and rotating the support members 110 into the respective threaded receiving apertures 134. It is also contemplated that the support members 110 and the imaging element 130 may be attached with a press or interference fit, a spline configuration, or a bayonet-type connection. It should be noted that support members 110 may be considerably more slender than than depicted, for example the members may be 2-5mm in diameter allowing for small incisions or needle like placement of the members. Imaging element 130 may be placed through a separate larger incision or trocar intended as the primary surgical incision. Thus, a separate incision for visualization is not necessary and the risk of incisional hernia is reduced..

Referring to FIGS. 3A-3C, insertion of a support member 110 into a position control member 120 and the subsequent locking in position of a support member 110 is shown. As seen in the cross-sectional view of FIG. 3A, position control member 110 may include a rotatable element 126, which rotates, pivots or otherwise moves within receiving portion 122. Rotatable element 126 may be seated in position control member 120 in a ball-and-socket configuration. Other configurations are contemplated to allow support members 110 to pivot within position control members 120, such as a movable structure of rods, levers, and/or cuffs. Rotatable element 126 may also extend into the position control member 120 below the receiving portion 122. Rotatable element 126 includes a passage 126a therethrough that aligns with the passage 120d through the receiving member 122. Adjacent to the rotatable member 126 is locking mechanism 124, which is shown as a screw 124a that advances through a threaded passage 122a in a portion of receiving member 122. Other locking mechanisms are contemplated for use in the position control member 120, such as a knob or button lock, or a clamping collar.

Turning now to FIG. 3B, a support member 110 is shown partially inserted through the passage 120d and the passage 126a. Rotatable member 126 allows for support member 110 to pivot within position control member 120. As the support member 110 is free to translate through the passages 120d, 126a and pivot relative to the passage 120d, the position control member 120 is in an unlocked position, as shown with the screw 124a backed out of the threaded passage 122a. With the screw 124a spaced from the rotatable member 126 and support member 110, the screw 124a does not inhibit the motion of support member 110 through the position control member 120.

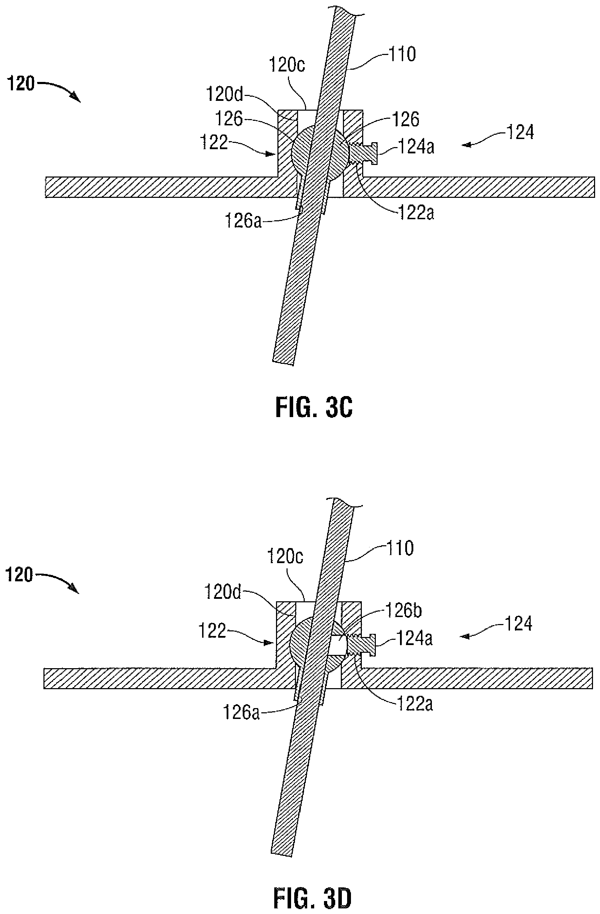

Referring to FIG. 3C, the position control member 120 is shown in a locked position, with the screw 124a advanced through the threaded passage 122a and in contact with the rotatable member 126. As the screw 124a frictionally engages the outer surface of rotatable member 126, support member 110, inserted therethrough, is prevented from further pivoting within the passage 120d. The rotatable member 126 may exert a compressive force on the support member 110 disposed through the passage 126a, inhibiting it from further translating axially through the position control member 120.

As shown in FIG. 3D, the rotatable member 126 may also include a passage 126b through which the screw 124a may advance to directly contact the support member 110. The screw 124a is advanced through passage 126b until direct contact with support member 110 is made, and maintains the desired depth of insertion of the support member 110 through the passages 120d, 126a. The screw 124a also prevents the support member 110 from pivoting relative to the position control member 120 by bracing it against the passages 120d, 126a.

Alternatively, the passage 126a may be dimensioned such that the support member 110 frictionally engages the rotatable member 126 upon insertion. The frictional engagement of the support member 110 and rotatable member 126 ensure that the support member is maintained at a desired depth in the body cavity 500b. The axial depth of the support member 110 may be changed by additional forces exerted by an operator. Still further, position control member 120 may incorporate a separate locking mechanism to inhibit the axial translation of support member 110 (not shown).

Turning now to FIG. 4, a full assembly view of surgical access assembly 100 is shown. In use, an operator will place position control members 120 on a body surface 500a to designate the points of insertion for the support members 110 through a tissue layer 500. The support members 110 are then inserted into the respective position control members 120, through the tissue layer 500, and into a body cavity 500b. Support members 110 are positioned to a desired axial depth and pivoted to a desired degree within position control members 120. Imaging member 130 is inserted through the layer of tissue 500 either through a separate incision, or an appropriately sized incision through which one of the support members 110 may also be inserted. Graspers, forceps, or other instruments may be used to hold the imaging member 130 in place in the body cavity 500b prior to assembly of the surgical support assembly 100. The threaded distal portions 110a of the support members 110 are then attached to the threaded receiving apertures 134 of the imaging member 130. With the imaging member 130 positioned above the surgical site in the internal body cavity 500b, the position control members 120 are locked such that the support members 110 are maintained in their desired axial and angular positions. Locking of the position control members 120 is accomplished by advancing the set screw 124a through the threaded passage 122a. The imaging aperture 132 of the imaging member 130 is then securely positioned to collect images from the surgical site below. When minimally invasive procedures are completed, the position control members 120 are unlocked, and the support members may be detached from the imaging member 130 and removed from the tissue layer 500 and the position control members 120. The imaging member 130 then may also be removed from the body cavity 500b and the position control members 120 removed from the body surface 500a.

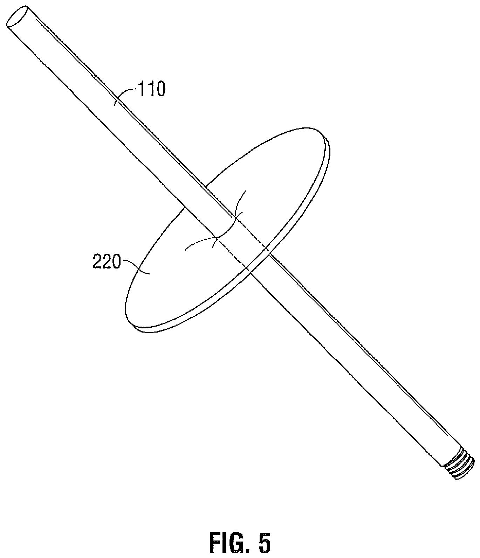

Referring to FIG. 5, an embodiment of a position control member 220 is shown with a support member 110 inserted therethrough. Position control member 220 is shown as a flat pad to be disposed on a body surface 500a. Any number of shapes and layered configurations are contemplated for position control member 220. Position control member 220 may additionally be treated with an adhesive on its underside 220b (not shown) to aid in the attachment of position control member 220 to a body surface 500a (not shown). Position control member 220 is formed of a material suitable to frictionally engage an outer surface of support member 110 when the support member 110 is inserted therethrough, such as fabric or rubber. The frictional engagement of position control member 220 and support member 110 allows an operator to position the support member 110 at a desired depth, without unintended axial translation of the support member 110. This position control also maintains the angular position at which the support member 110 is inserted though the layer of tissue 500. Position control member 110 may be formed of various thicknesses to provide a desired degree of position control.

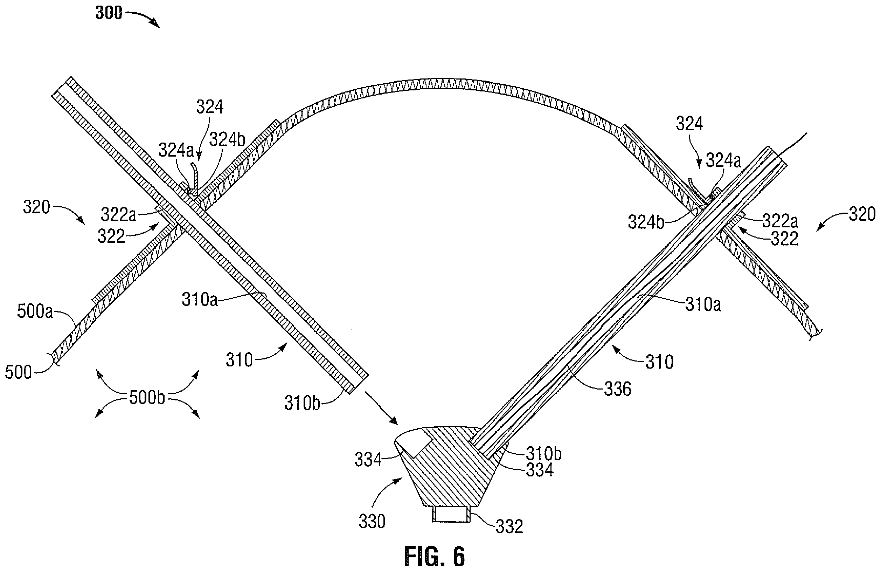

Turning now to FIG. 6, a partial assembly view of an embodiment of a surgical access assembly 300 is shown. Surgical access assembly 300 includes at least two support members 310. Support members 310 are substantially similar to support members 110 of the previous embodiments, but support members 310 contain a passage 310a extending the length of support member 310 through which equipment such as wires 336 may be passed. Wires may supply power or control signals to an imaging element 330 or may send or receive images between the imaging element 330 and another device.

Surgical access assembly 300 also incorporates position control members 320. Position control members 320 are substantially similar to position control members 120 discussed above, but do not include a rotatable member. Position control members 320 are disposed on a body surface 500a such that the support members 110 are inserted at desired angles relative to a body cavity 500b. Surgical access assembly 300 may be utilized on surgical sites with uneven or irregular body surface geometries. Position control members 320 include a receiving portion 322 extending from the flat profile of the position control member 320. The receiving portion 322 defines a passage 322a through which the support member 310 is inserted. A support member 310 may frictionally engage a position control member 320, or may be free to translate within the passage 322a. A latching member 324 may be present to lock the support member 310 in position in the position control member 320. The latching member 324 is a lever that rotates and causes an end 324b to frictionally engages an outer surface of the support member 310.

Surgical access assembly 300 includes imaging member 330. Imaging member 330 includes an imaging aperture 332 and receiving apertures 334. Distal portions 310b of support members 310 frictionally fit within receiving apertures 334. Other couplings between support members 310 and imaging member 330 are contemplated, including a threaded connection, tongue-and-groove connection, or bayonet-type coupling. With the support members 310 securely attached to the imaging member 330, the imaging aperture 332 faces body cavity 500b such that it can collect images from a surgical site below.

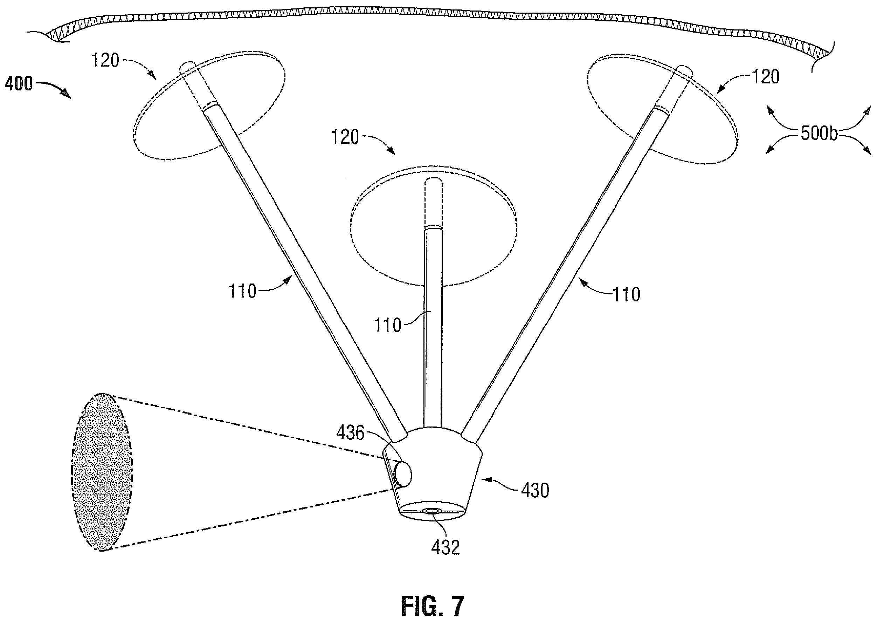

Referring to FIG. 7, an embodiment of a surgical access assembly 400 is shown. Surgical access assembly 400 incorporates support members 110, position control members 120, and an imaging member 430. Imaging member 430 incorporates an imaging aperture 432, and an image projecting element 436. Image projecting element 436 projects an image collected from imaging aperture 430 onto a portion of body cavity 500b. The projected image may be projected laterally, proximally or distally to display on any suitable surface in the body cavity 500b such as a tissue wall, on an implant, such as a hernia mesh or on a separate screen inserted into the body cavity 500b (not shown). The projected image allows an operator or surgeon to view the received image from a surgical site in situations where the field of view for an operator might be obscured, but where the image receiving element 432 is able to access. Alternatively, the image projecting element 436 may project images other than those from the image receiving element 432, such as images stored in memory or those externally transmitted to the imaging element 430. For example, he projected image may provide a target or project a preplanned template for an eventual configuration of tissue or project a go/no-go region.

In further embodiments, equipment such as wires or fiber optic cables may serve as position control members, and suspend an imaging member above a body cavity for use during minimally invasive procedures. In still further embodiments, a position control member may be inserted into a body cavity, extend through the body cavity, and exit a portion of the body cavity, with an imaging member mounted to the portion of the position control member mounted in the body cavity.

It will be understood that various modifications may be made to the embodiments disclosed herein. Therefore, the above description should not be construed as limiting, but merely as exemplifications of embodiments. Those skilled in the art will envision other modifications within the scope of the claims.

## Claims

1. A surgical support assembly, comprising:
a plurality of tissue penetrating support members (110);
and an imaging member (130) attached at the vertex of the plurality of tissue penetrating support members (110), wherein the tissue supporting members (110) are adjustably positionable and the surgical support assembly further comprises a plurality of position control members (120),
wherein each tissue penetrating support member (110) is insertable through a position control member; and **characterized by** each position control member (120) being adapted to allow translating and pivoting of the corresponding tissue penetrating support member (110) relative to the position control member and being operable to lock a corresponding tissue penetrating support member (110) at a desired position relative to the position control member (120),
such that the imaging member (130) is supported by the plurality of tissue penetrating support members (110) at a desired position.

2. The surgical support assembly of claim 1, wherein at least one of the tissue penetrating support members (110) is a rod.

3. The surgical support assembly of claim 1 or claim 2, wherein at least one of the plurality of tissue penetrating support members (110) has a passage therethrough.

4. The surgical support assembly of claim 3 wherein objects are inserted through the passage.

5. The surgical support assembly of any preceding claim, wherein at least one of the plurality of position control members (120) includes a rotatable member through which the tissue penetrating support member (110) is inserted.

6. The surgical support assembly of any preceding claim, wherein at least one of the plurality of position control members (120) includes a lock for maintaining the position of a tissue penetrating support member (110) relative to the position control member (120).

7. The surgical support assembly of any preceding claim, wherein the imaging member (130) includes a camera.

8. The surgical support assembly of any preceding claim, wherein the imaging member (130) includes a projector.

9. The surgical support assembly of any preceding claim, wherein the imaging member (130) includes an aperture (134) for receiving a tissue penetrating support member (110).

10. The surgical support assembly of claim 1, wherein the at least one tissue penetrating support member (110) is adjustably positionable through a position control member at an angle relative to the position control member (120)

## Patentansprüche

1. Chirurgische Stützanordnung, die umfasst:
eine Vielzahl von gewebepenetrierende Stützelementen (110);
und ein bildgebendes Element (130), das an dem Scheitelpunkt der Vielzahl von gewebepenetrierenden Stützelementen (110) befestigt ist,
wobei die gewebepenetrierenden Stützelemente (110) justierbar positionierbar sind und die chirurgische Stützanordnung des Weiteren eine Vielzahl von Positionssteuerelementen (120) umfasst,
wobei jedes gewebepenetrierende Stützelement (110) durch ein Positionssteuerelement einführbar ist; und **dadurch gekennzeichnet ist, dass**
jedes Positionssteuerelement (120) angepasst ist, um Übersetzen und Schwenken der entsprechenden gewebepenetrierenden Stützelemente (110) relativ zu dem Positionssteuerelement zu ermöglichen, und betreibbar ist, um ein entsprechendes gewebepenetrierendes Stützelement (110) an einer gewünschten Position relativ zu dem Positionssteuerelement (120) zu verriegeln,
derart, dass das bildgebende Element (130) durch die Vielzahl von gewebepenetrierenden Stützelementen (110) an einer gewünschten Position gestützt wird.

2. Chirurgische Stützanordnung nach Anspruch 1, wobei mindestens eines der gewebepenetrierenden Stützelemente (110) eine Stange ist.

3. Chirurgische Stützanordnung nach Anspruch 1 oder Anspruch 2, wobei mindestens eines der Vielzahl von gewebepenetrierenden Stützelementen (110) eine Durchführung dahindurch hat.

4. Chirurgische Stützanordnung nach Anspruch 3, wobei Objekte durch die Durchführung eingeführt werden.

5. Chirurgische Stützanordnung nach einem der vorstehenden Ansprüche, wobei mindestens eines der Vielzahl von Positionssteuerelementen (120) ein drehbares Element umfasst, durch welches das gewebepenetrierende Stützelement (110) eingeführt wird.

6. Chirurgische Stützanordnung nach einem der vorstehenden Ansprüche, wobei mindestens eines der Vielzahl Positionssteuerelementen (120) eine Verriegelung zum Aufrechterhalten der Position eines gewebepenetrierenden Stützelements (110) relativ zu dem Positionssteuerelement (120) umfasst.

7. Chirurgische Stützanordnung nach einem der vorstehenden Ansprüche, wobei das bildgebende Element (130) eine Kamera umfasst.

8. Chirurgische Stützanordnung nach einem der vorstehenden Ansprüche, wobei das bildgebende Element (130) einen Projektor umfasst.

9. Chirurgische Stützanordnung nach einem der vorstehenden Ansprüche, wobei das bildgebende Element (130) eine Öffnung (134) zum Aufnehmen eines gewebepenetrierenden Stützelements (110) umfasst.

10. Chirurgische Stützanordnung nach Anspruch 1, wobei das mindestens eine gewebepenetrierende Stützelement (110) durch ein Positionssteuerelement in einem Winkel relativ zu dem Positionssteuerelement (120) justierbar positionierbar ist.

## Revendications

1. Ensemble support chirurgical, comprenant :
une pluralité d'éléments supports de pénétration tissulaire (110) ;
et un élément d'imagerie (130) fixé au sommet de la pluralité d'éléments supports de pénétration tissulaire (110),
dans lequel les éléments supportant les tissus (110) peuvent être positionnés de manière réglable et l'ensemble support chirurgical comprend en outre une pluralité d'éléments de commande de position (120),
dans lequel chaque élément support de pénétration tissulaire (110) peut être inséré à travers un élément de commande de position ; et **caractérisé par**
chaque élément de commande position (120) étant conçu pour permettre une translation et un pivotement de l'élément support de pénétration tissulaire correspondant (110) par rapport à l'élément de commande de position et pouvant fonctionner pour verrouiller un élément support de pénétration tissulaire correspondant (110) en une position souhaitée par rapport à l'élément de commande de position (120),
de sorte que l'élément d'imagerie (130) soit supporté par la pluralité d'éléments supports de pénétration tissulaire (110) en une position souhaitée.

2. Ensemble support chirurgical selon la revendication 1, dans lequel au moins l'un des éléments supports de pénétration tissulaire (110) est une tige.

3. Ensemble support chirurgical selon la revendication 1 ou la revendication 2, dans lequel au moins l'un de la pluralité d'éléments supports de pénétration tissulaire (110) a un passage le traversant.

4. Ensemble support chirurgical selon la revendication 3, dans lequel des objets sont insérés à travers le passage.

5. Ensemble support chirurgical selon l'une quelconque des revendications précédentes, dans lequel au moins l'un de la pluralité d'éléments de commande de position (120) inclut un élément rotatif à travers lequel l'élément support de pénétration tissulaire (110) est inséré.

6. Ensemble support chirurgical selon l'une quelconque des revendications précédentes, dans lequel au moins l'un de la pluralité d'éléments de commande de position (120) inclut un verrou pour maintenir la position d'un élément support de pénétration tissulaire (110) par rapport à l'élément de commande de position (120).

7. Ensemble support chirurgical selon l'une quelconque des revendications précédentes, dans lequel l'élément d'imagerie (130) inclut une caméra.

8. Ensemble support chirurgical selon l'une quelconque des revendications précédentes, dans lequel l'élément d'imagerie (130) inclut un projecteur.

9. Ensemble support chirurgical selon l'une quelconque des revendications précédentes, dans lequel l'élément d'imagerie (130) inclut une ouverture (134) pour recevoir un élément support de pénétration tissulaire (110).

10. Ensemble support chirurgical selon la revendication 1, dans lequel l'au moins un élément support de pénétration tissulaire (110) peut être positionné de manière réglable à travers un élément de commande de position selon un angle par rapport à l'élément de commande de position (120).
